# EUROPEAN PATENT APPLICATION

(11) **EP 3 572 808 A1**
(43) Date of publication of application: **27.11.2019**
(21) Application number: 18174067.1
(22) Date of filing: 24.05.2018
(51) Int. Cl.: G01N 33/00, G01M 15/10, G01N 1/22

(54) **EMISSION MEASUREMENT SYSTEM FOR TESTING A COMBUSTION ENGINE INCLUDING EXHAUST GAS EXPOSURE OF BIOLOGICAL CELLS**

(71) Applicant: FRAUNHOFER-GESELLSCHAFT zur Förderung der angewandten Forschung e.V., 80686 München (DE)
(72) Inventor: ZIMMERMAN, Heiko, 97295 Waldburn (DE); VON BRIESEN, Hagen, 66280 Sulzbach (DE); WILHELM, Nadine, 66280 Sulzbach (DE); WAGNER, Sylvia, 66280 Sulzbach (DE); KOHL, Yvonne Lydia, 66280 Sulzbach (DE)
(74) Representative: v. Bezold & Partner Patentanwälte - PartG mbB

(57) **Abstract**

An emission measurement system 100 for testing a combustion engine 11, in particular of a vehicle 10, comprises a sampling device 20 for collecting exhaust gas 12 created by the combustion engine 11 and a testing device 30 being coupled with the sampling device 20 and being configured for applying at least one test to a test sample 13 including the exhaust gas 12, wherein the testing device 30 includes a cell exposure device 31 being configured for accommodating at least one sample 1 of biological cells 2 and exposing the biological cells 2 to the test sample 13 and an analysing device 33 being configured for testing the biological cells 2. The emission measurement system 100 comprises e. g. a car test bench or portable test apparatus. Furthermore, methods of testing a combustion engine 11, in particular of a vehicle 10, are described.

## Description

The present invention relates to emission measurement systems and methods for testing a combustion engine, e. g. of a vehicle, by collecting exhaust gas from the combustion engine and applying at least one test to the exhaust gas. Applications of the invention are available in particular in the fields of exhaust gas testing with combustion engines of vehicles or other crafts or machines.

Exhaust gas emissions from combustion engines represent an increasing issue in all fields of operating combustion engines, e. g. in vehicles, ships, aircrafts or other machines. The exhaust gas (or: exhaust smoke) includes toxic gases and/or particles, which may have a harmful effect on biological organisms, in particular humans and animals, after inhalation of the gas emissions or application on the skin. Roller type car test stands are generally known which allow an analysis of the exhaust gas composition. However, the information obtained on the contents of the exhaust gas is not sufficient for investigation the effect on biological organisms. It is known that some automotive companies have conducted exhaust gas *in vivo* tests with monkeys in the past as reported in public media. Needless to say, these *in vivo* tests are not acceptable for ethical reasons.

Tests methods for analysing effects of individual gases or aerosols on biological organisms by *in vitro* tests are generally known. As an example, P. S. Hiemstra et al. (Toxicology in Vitro 47 (2018) 137-146) have described the use of human lung epithelial cell cultures for analysis of inhaled toxicants. In gas supply set-ups for evaluating the cellular effects of toxicants, a test atmosphere is generated by a gas or aerosol generator, optionally diluted and then supplied to a cell culture in dishes or a microfluidic system. As a disadvantage, these tests usually have a limited significance as they are conducted at laboratory conditions. The gas supply set-ups are not adapted for operation under practical conditions of an environmental or technical source of toxicants. The cell cultures subjected to the gas and/or aerosol need to be analysed in a separate equipment. The test results obtained usually are not sufficient for characterizing the real effect of toxicant emissions, so that the conventional laboratory techniques have only limited applications. As a further disadvantage, a duration of cell exposure beyond some hours is a limitation with the conventional *in vitro* test systems.

The objective of the invention is to provide an improved emission measurement system, being configured for testing a combustion engine, e. g. of a vehicle, being capable of avoiding limitations of conventional techniques. In particular, the emission measurement system is to be capable of testing the effect of exhaust gas on biological organisms under practical operations conditions of a combustion engine emitting the exhaust gas and/or with a compact system design and/or with a broad range of applicable analysing procedures.

According to a first general aspect of the invention, the above objective is solved by an emission measurement system for testing a combustion engine, e. g. of a vehicle, comprising a sampling device for collecting exhaust gas created by the combustion engine and a testing device being coupled with the sampling device. The combustion engine (or: combustion motor) is any engine which is adapted for a combustion of a fuel, e. g. for directly or indirectly generating mechanical power. The term combustion engine also covers supplementary components, like a catalytic converter. The exhaust gas is produced by the fuel combustion (in particular gas or diesel or aviation turbine fuel combustion) and optionally passed through the catalytic converter. The term exhaust gas refers to any gaseous or vaporous fluid emitted by the engine, including gas, vapour and/or particles, like aggregates or particles of combustion products. The engine includes an exhaust output (e. g. exhaust pipe) through which the exhaust gas is emitted from the engine. With preferred applications of the invention, the combustion engine is a driving motor or generator included in a vehicle, other craft or machine.

The sampling device comprises an inlet duct to be coupled with the combustion engine, preferably with the exhaust output thereof, an exhaust gas guiding system and at least one outlet duct coupled with the testing device. The inlet duct can be arranged for accommodating the complete exhaust gas flow emitted by the engine or for branching a portion thereof. The exhaust gas guiding system is arranged for matching conditions of the exhaust gas to the test to be applied in the testing device, and it includes at least one line between the inlet and outlet ducts.

The testing device is adapted for applying at least one test to a test sample (or: exhaust gas sample or gas/smoke sample) including the exhaust gas. The test sample is a quantity of gas, consisting of the exhaust gas as emitted by the combustion engine or consisting of a mixture of the exhaust gas with a dilution gas, typically air. Applying the at least one test to the test sample generally comprises an interaction of the test sample with a sensor material and detecting a change of at least one parameter of the sensor material.

According to the invention, the testing device includes a cell exposure device for accommodating at least one sample of biological cells (cell sample) and an analysing device for testing the biological cells, in particular during and/or after exposure to the test sample. The at least one sample of biological cells provides the sensor material, to which the test sample is to be applied. The cell exposure device is configured for accommodating the biological cells with an exposed sample surface and for guiding the test sample to the biological cells, e. g. directing a flow of the test sample to the biological cells and/or creating a test sample atmosphere contacting the biological cells. The analysing device includes at least one measuring unit for detecting at least one read-out parameter of the cells. The analysing device can be fixedly coupled with the cell exposure device, so that advantages in terms of immediate cell testing are obtained. Alternatively, it can be provided as a separate unit, wherein the at least one sample of biological cells can be transferred from the cell exposure device to the analysing device after the exposure to the test sample. In this case, advantages in terms of the broad range of available tests can be obtained. By testing the cells, information of the effect of the test sample and thus the effect of the exhaust gas on biological organisms is obtained. The occurrence and/or degree of cell alterations are provided as an information on the effect of the exhaust gas.

According to a second general aspect of the invention, the above objective is solved by an exhaust gas testing method for testing a combustion engine, in particular of a vehicle, comprising the steps of coupling the combustion engine with an emission measurement system, operating the combustion engine and creating the exhaust gas, collecting the exhaust gas, and testing a test sample including the exhaust gas, wherein the test sample testing step includes exposing at least one sample of biological cells to the test sample and testing the biological cells. Testing the effect of the exhaust gas included in the test sample on the biological cells preferably includes determining, whether the biological cells are changed by the exposure to the test sample, and, in case of a change, characterizing the altered cells, determining a degree of change and/or determining a dependency of the change on engine operation conditions, like a duration of exposure or a power of engine operation. Preferably, the inventive method is conducted with the emission measurement system according to a first general aspect of the invention.

Advantageously, the invention provides an exhaust gas emission measurement for testing the effect of exhaust gas on biological cells, wherein the exhaust gas is collected from a combustion engine and applied to the cells during the operation of the engine. The exhaust gas is emitted with a temperature above the temperature of an environment of the combustion engine and with a predetermined flow rate from the combustion engine. The inventors have found that the combination of the sampling device and the cell exposure device allows an immediate interaction of the exhaust gas with the cells under practical operation conditions, while limitations of conventional laboratory systems can be overcome. In particular, it has been found, that sampling the exhaust gas allows a temperature and flow rate reduction. Thus, the sampling device, in particular the exhaust gas guiding system thereof, fulfils a double function in terms of (i) providing the test sample and (ii) conditioning the test sample such that it is supplied to the biological cells in a condition which corresponds to the exposure of biological organisms in an environment of the combustion engine, e. g. during inhalation or application on the skin.

Furthermore, it has been found that biological cells can be used as an organ model directly interacting with the exhaust gas test sample like cells of the same type in a complete organism. Thus the inventive emission measurement system provides significant results while avoiding the ethical problems of *in vivo* tests.

As a further advantage, the invention provides a compact system design by coupling the sampling and cell exposure. Advantageously, this allows the integration of the inventive emission measurement system into an available stationary or mobile test equipment. Another advantage of the invention results from a broad range of applicable analysing procedures

According to preferred applications of the invention, the emission measurement system is employed for testing the combustion engine of a vehicle, like a car or a truck. Advantageously, stationary or mobile emission measurement systems are available. The stationary emission measurement system (first embodiment of the invention) is configured for an operation at a fixed location, wherein the vehicle is not moved relative to a ground during operating the combustion engine. The mobile emission measurement system (second embodiment of the invention) is configured for coupling with the vehicle,

With the first embodiment, the emission measurement system preferably is a car test bench, e. g. a roller type car test stand, which is adapted for the stationary test operation. The car test bench includes a roller module for accommodating the vehicle. Advantageously, the invention can be integrated into available test equipment. The roller module can be a stationary module integrated in a test stand or a mobile module provided at the location of testing the combustion engine, e. g. in a workshop.

According to an advantageous variant of the first embodiment, the emission measurement system can be provided with an exhaust gas container which is configured for coupling with an exhaust output of the combustion engine of the vehicle. In this case, the sampling device can be coupled with or provided by the exhaust gas container. The exhaust gas container, preferably having a volume of more than 1 m³, e. g. a volume in a range from 30 m³ to 70 m³ (like a standard transport container), has advantages for creating an atmosphere similar to environmental conditions, e. g. along roads. Preferably, the exhaust gas container has gas-tight walls. Thus, the collecting step of the inventive method can include at least one of collecting the exhaust gas at the exhaust output of the combustion engine or in the exhaust gas container coupled with the exhaust output of the combustion engine.

With the second embodiment, the emission measurement system preferably is a portable test apparatus being configured for a mobile test operation. The portable test apparatus includes a carrier device being configured for coupling with the vehicle. The carrier device comprises e. g. a support platform coupled with the vehicle or a part of the vehicle itself. In other words, the emission measurement system can be arranged outside or inside the vehicle during the driving operation thereof. Advantageously, with the mobile embodiment, cell exposure to the test sample can be obtained during real operation conditions of the vehicle, e. g. during different operation phases, like driving in town traffic or on a highway, velocity cycles .

According to a further preferred embodiment of the invention, the sampling device is adapted for direct coupling with an exhaust output of the combustion engine. The inlet duct of the sampling device has a pipe coupler, like a bracket, clamp and/or shell, for positioning an opening of the inlet duct in the flow of exhaust gas. The opening of the inlet duct is dimensioned for collecting all exhaust gas or a portion thereof.

The sampling device includes an exhaust gas guiding system which in the simplest case includes a single or branched duct line between the inlet and outlet ducts of the sampling device. Depending on the application of the invention, the exhaust gas guiding system can include further conditioning components arranged along the duct line for providing and conditioning the test sample. Preferably, at least one of the following components can be provided. According to a first variant, the sampling device includes a metering device for setting and supplying a predetermined test volume of the test sample. Thus, advantages for adjusting predetermined test conditions by setting the test sample volume are obtained. The test sample volume can be set e. g. in dependency on preparing tests or experimental reference data. According to a second variant, the sampling device includes a dilution device for mixing the collected and optionally metered exhaust gas with a test gas, preferably air, so that the test sample is provided with a predetermined exhaust gas concentration. Advantageously, the exhaust gas concentration in the test sample can be set according to test conditions to be obtained. As an example, the exhaust gas concentration can be set for simulating an exhaust gas exposure, e. g. directly on a road, at a roadside, in a vehicle and/or in a house. According to a third variant, the sampling device includes a temperature control unit being adapted for setting a test temperature of the test sample equal to a physiological temperature, e. g. 37 °C, or to a predetermined stimulus temperature below the physiological temperature, e. g. in a range down to 0 °C, or above the physiological temperature, e. g. in a range up to 50 °C. Setting the physiological temperature has advantages for testing the effect of the test sample at natural conditions, while setting the stimulus temperature can result in an increased sensitivity of the test. As an example, the stimulus temperature below the physiological temperature can induce an inflammation stimulus as it would influence an asthmatic. Preferably, the temperature control unit is arranged at a downstream end of the exhaust gas guiding system.

A further important advantage of the invention results from the fact, that the at least one sample of the biological cells provided in the cell exposure device can be configured in dependency on the particular test to be conducted. Generally, the at least one sample of the biological cells is provided in a condition separated from a cell donor, e. g. a human or an animal. Preferably, the biological cells comprise at least one of a cell culture and cell tissue, e. g. in a culture dish or another receptacle, adapted for the subsequent analysis, like a multiwell plate. The cells preferably include at least one of stem cells, in particular induced pluripotent stem (ips) cells, differentiated ips cells, in particular ips lung cells and/or ips epithelial cells, 2D cell cultures, 3D cell cultures, cell organoids, a plurality of different genetic variants of the biological cells, primary lung cells, in particular from lung tissue taken from a human or an animal, and epithelial cells, in particular from epithelial tissue taken from a human or an animal. Different cell types can be combined as cultures and/or tissue in one or more cell samples in the cell exposure device.

According to a further advantageous embodiment of the invention, the cell exposure device can be configured for accommodating the biological cells in a frozen state and thawing the biological cells before exposing them to the test sample. Advantageously, the test duration can be extended by providing fresh thawed cells immediately at the beginning of the test sample exposure and/or by reloading fresh thawed cells at predetermined intervals during the test.

Further advantageous features of preferred embodiments of the invention relate to the configuration of the analysing device, in particular according to at least one of the following variants. Firstly, the analysing device can be configured for testing the biological cells during and/or after exposing them to the test sample. Testing the biological cells during the test sample exposure comprises measurements with the cells directly in the cell exposure device, such as optical or electrical measurements, e. g. for detecting the occurrence of cell changes or a cell apoptosis. Testing the biological cells after the test sample exposure comprises measurements with the cells taken from the cell exposure device, e. g. with single cells taken from the cell exposure device during a running test sample application or with a complete sample of biological cells. Furthermore, the analysing device can be configured for measuring the introduction of particles into the biological cells. To this end, the analysing device can include a microscope device for direct or subsequent observation of cells in the cell exposure device or taken therefrom. If the analysing device is configured for detecting cell apoptosis, particular advantages for an early detection of dangerous exhaust gases are obtained.

Generally, the analysing device can be adapted for testing the cells according to available read-outs for cell characterization, e. g. as summarized by P. S. Hiemstra et al. (cited above). In particular, the analysing device can be adapted for detecting at least one of the read-outs including necrosis, apoptosis, miRNA pattern, epigenetic alterations, cell death, DNA breaks, miRNA expression, keeping a cell culture barrier function, cytokine release, ciliary activity (for airway epithelial cells), mucus viscosity alteration, mucus hypersecretion, cell metabolism, markers of oxidative stress, induction of an oxidative stress response and inflammatory response, induction of an integrated stress response, impairment of autophagy, impairment of epithelial antimicrobial defence, adsorption of test compounds, epithelial differentiation after repeated exposures, engineered reporter cell lines, e. g. iPSC single or multiple reporter lines. As a particular example, polycyclic aromatic hydrocarbon (PAH) exposure can be detected by an increased DNA methylation at several CpG sites.

According to a further advantageous embodiment of the invention, the step of operating the combustion engine, e. g. of a vehicle, includes setting of different operation phases of the vehicle, and the steps of collecting the exhaust gas and testing the test sample can be repeated in each of the operation phases of the engine. The operation phases can differ e. g. with regard to an output power of the engine or other operation conditions of the engine, like a setting of a catalytic converter. Advantageously, this facilitated a complete characterization of the engine and the recognition of possible dangerous operation modes of the engine.

Further advantages of the invention are summarized as follows. Advantageously, the emission measurement can be combined with available cell handling procedures, like bio-banking, automated expansion of cultures and differentiation and/or high-throughput screening. The cell sample can be exposed to the exhaust gas test sample, in particular at the air-liquid interface (ALI) under realistic conditions *in vitro.*

Furthermore, the invention allows a risk assessment of exhaust gas effects, like the toxicology (e. g. detecting apoptosis, stress pathways and/or necrosis) and the genotoxicology (e. g. detecting DNA breakage, chromosomal abnormalities and/or epigenetic alterations) thereof. To this end, the emission measurement can be combined with a physical and/or chemical analysis of the exhaust gas, in particular for determining physicochemical properties, a chemical composition, particle corona, particle contents and/or gas contents of the exhaust gas. As an example, exhaust gas from a gas engine includes nitrogen oxides (nitrogen oxide, nitrogen dioxide), hydrocarbons like polycyclic aromatic hydrocarbons (PAH) and carbon monoxide. As a further example, exhaust gas from a diesel engine includes nitrogen oxides (nitrogen oxide, nitrogen dioxide), hydrocarbons like PAH, carbon monoxide and diesel exhaust particles (DEP). The DEP may have different particle features, like shapes, sizes, surface reactivity (charge, reactive groups) and adherence of different components, in particular including a carbon core which adsorbs mixture of metals and organic chemicals (including carcinogens). DEP sizes comprise e. g. particulate matter <10 µm (travelling through the nasal cavity into deeper areas of the bronchial tubes), particles <2,5 µm (penetrating into the bronchioles and alveoli) and ultrafine particles smaller than 0,1 µm (getting into the lung tissue and the bloodstream).

The invention allows population studies, including investigating patient-specific cells (e. g. smokers vs. non-smokers, rural area vs. urban area, familial lung cancer, prenatal exposure), allergic disorders, environmental exposures during pregnancy and/or a combination of environmental and genetic factors.

Further details and advantages of the invention are described in the following with reference to the attached drawings, which show in:
- Figure 1:: a schematic illustration of the first embodiment of the invention, including a stationary emission measurement system;
- Figure 2:: a schematic illustration of a modified variant of the first embodiment of the invention, including a stationary emission measurement system;
- Figure 3:: a schematic illustration of the second embodiment of the invention, including a mobile emission measurement system;
- Figure 4:: a schematic illustration of a sampling device included in an emission measurement system of the invention; and
- Figure 5:: a schematic illustration of a testing device included in an emission measurement system of the invention.

Features of preferred embodiments of the invention are described in the following with exemplary reference to an emission measurement system adapted for testing vehicle engines. The invention is not restricted to this application. The invention correspondingly can be applied for testing other engines, e. g. of other crafts or machines. Furthermore, reference is made to the configuration of the emission measurement system for engine tests. Details of procedures for preparing (in particular culturing) and/or analyzing the cells are not described, as those procedures are known per se from prior art. In particular, for analyzing the cells, standard conditions can be employed as they are known from available procedures.

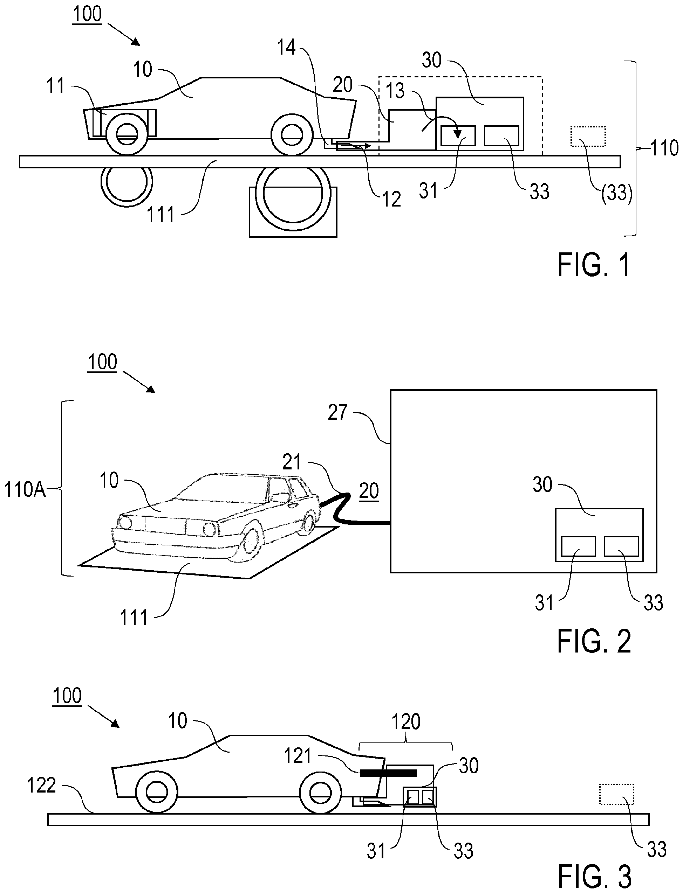

Figure 1 schematically shows the first embodiment of the invention with a stationary emission measurement system 100, being configured as a car test bench 110 with a roller module 111 accommodating a vehicle 10, a sampling device 20 and a testing device 30. The car test bench 110 with the roller module 111 is designed as it is known from conventional test stands. Further to the illustrated devices, a cell sample preparation device and/or a main control device (not shown) can be provided. The cell sample preparation device includes components for preparing a cell culture or tissue before arranging it in the testing device 30. The control device can be coupled with some or all of the components 110, 20 and/or 30 and/or with the vehicle 10.

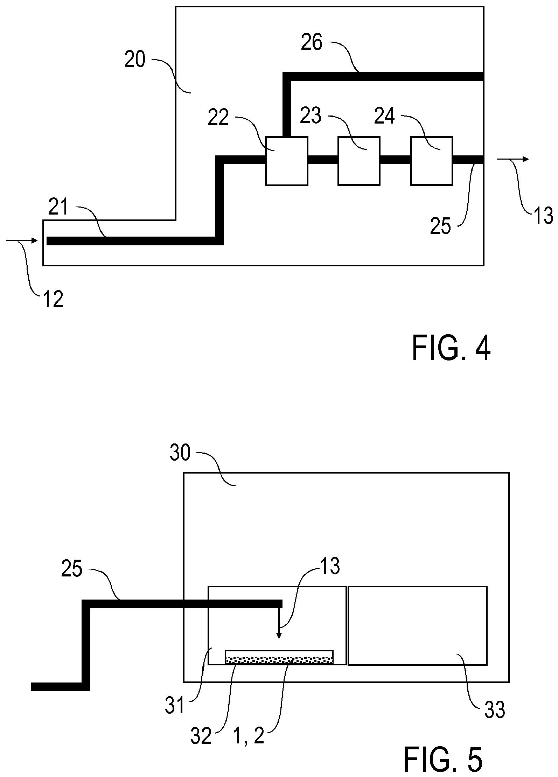

The sampling device 20 is coupled with the exhaust output 14 of the vehicle 10. When the combustion engine 11 is operated on the roller module 111, exhaust gas is produced, while the vehicle 10 is kept in place. A flow of the exhaust gas (see arrow 12) is collected by the sampling device 20, the details of which are described below with reference to Figure 4.

The testing device 30 is coupled with the sampling device 20. At least one test sample (see arrow 13) including the exhaust gas 12 is fed to a cell exposure device 31 including at least one sample 1 of biological cells 2 (see Figure 5). The test sample 13 flows to the biological cells, which are altered in dependency on the occurrence of toxicants in the test sample 13. Changes of the cells are detected with an analysing device 33, which is arranged as a component of the testing device 30 or as a separate component, e. g. in a separate room of the car test bench 110.

For testing the combustion engine 11, the vehicle 10 is operated, exhaust gas 12 is collected, a test sample 13 is provided, the cells are exposed to the test sample 13, and changes of the cells are analysed with the analysing device 33. This can be done with a continuous test, while the vehicle 10 is operated with different operation conditions, or with interrupted, step-wise tests, wherein the conditioning of the test sample can be changed in dependency on a previous test result. The analysing device 33 provides at least one read-out parameter of the cells, like e. g. a degree of apoptosis or a degree of DNA methylation.

Generally, the read-out parameter(s) can be used as the result of the emission measurement already, in particular for characterizing the effect of the exhaust gas and/or the engine. Optionally, the emission measurement can be combined with a physical and/or chemical characterization of the exhaust gas obtained by an additional analysis of the exhaust gas and/or reference information. Testing the cells can be combined with an exhaust gas collection, in particular a test sample collection, and a physical and/or chemical analysis of the exhaust gas. The result of the physical and/or chemical analysis, in particular the chemical composition and particle features are output in combination with the read-out parameter(s) obtained from the cell analysis. Subsequently, a risk assessment of the particularly tested exhaust gas and/or engine, a population analysis and/or data on a correlation of exhaust gas composition and exhaust gas effect, like e. g. toxicology, can be included for providing the result of the emission measurement.

Figure 2 shows a modified variant of the first embodiment of the invention with a stationary emission measurement system 110A, wherein the sampling device 20 includes an exhaust gas container 27, including at least the cell exposure device 31 of the testing device 30. When the vehicle 10 is operated on the roller module 111, exhaust gas is supplied via the inlet duct 21 of the sampling device 20 into the exhaust gas container 27, where it is distributed like in an environment. The cell exposure device 31 is arranged at the bottom of the exhaust gas container 27 or in a certain height above the bottom, thus allowing an investigation of exhaust gas effects, in particular particle effects, in dependency on the height above ground.

The second embodiment of the invention, including a mobile emission measurement system 120 is shown in Figure 3. The mobile emission measurement system 120 includes a carrier device 121, like a support beam or a platform accommodating the sampling device 20 and the testing device 30 (at least the cell exposure device 31 thereof). When the vehicle 10 is operated on the ground 122, e. g. a road, the inventive test is conducted by collecting exhaust gas and applying it to the cells in the cell exposure device 31. The analysing device 33 is integrated in the mobile emission measurement system 120, or it can be arranged separately for analysing cell changes after driving the vehicle 10.

According to Figure 4, the sampling device 20 comprises the inlet duct 21 to be coupled, e. g. via a probe head or as a complete tube connection, with the exhaust opening of the vehicle for collecting the exhaust gas 12. The inlet duct 21 branches into the outlet duct 25 for supplying a test sample 13 to the cell exposure device 31 and an exhaust duct 16 through which exhaust gas is discharged to the surrounding. A metering device 22, like a first valve, is provided for adjusting the volume of exhaust gas to be tested. At the downstream side of the metering device 22, a dilution device 23, like a second valve is arranged for mixing the volume of exhaust gas with air and the setting a volume concentration of the exhaust gas in the test sample. The air is supplied e. g. from the surrounding or from a reservoir (not shown). Finally, the test sample flows through a temperature control unit 24 setting the temperature of the test sample to a physiological temperature. The temperature control unit 24 includes a temperature sensor (not shown). The output signal of the temperature sensor is used for a loop control of the test sample temperature. The components 22, 23 and 24 provide an exhaust gas guiding system for conditioning the test sample. They are controllable with the main control device mentioned above. Depending on the design of the ducts and the test conditions, one or more of the components 22, 23 and 24 can be omitted.

Figure 5 schematically illustrates details of the testing device 30, including the cell exposure device 31 and the analyzing device 33. The outlet duct 25 of the sampling device 20 opens into the cell exposure device 31. At least one sample receptacle 32 is arranged in the cell exposure device 31, including the sample 1 of biological cells 2. The biological cells 2 are exposed in the sample receptacle 32, i. e. preferably they are free of a covering cultivation liquid. The outlet duct 25 can be provided with a flow distributor for directing the test sample 13 towards the whole surface of the sample 1. The analyzing device 33 is configured in dependency on the test to be conducted at the cells 2. For example, the analyzing device 33 may comprise a microscope unit, a spectroscopic measurement device and/or an impedance measurement device for investigating the cells 2. For more complex read-outs, the analyzing device 33 may include a complex laboratory equipment, e. g. for detecting genetic alterations of the cells and/or for analyzing cell functions and/or metabolism.

The illustrated emission measurement systems 100 can be modified, e. g. by providing multiple outlet ducts and multiple samples to be exposed to test samples. As a further modification, the analyzing device and the cell exposure device can be an integrated component. Furthermore, additional known exhaust gas tests can be combined with the inventive cell-based test, e. g. a chemical exhaust gas analysis.

The features of the invention disclosed in the above description, the drawings and the claims can be of significance individually, in combination or sub-combination for the implementation of the invention in its different embodiments.

## Claims

1. Emission measurement system (100), being configured for testing a combustion engine (11), in particular of a vehicle (10), comprising
- a sampling device (20) being arranged for collecting exhaust gas (12) created by the combustion engine (11), and
- a testing device (30) being coupled with the sampling device (20) and being configured for applying at least one test to a test sample (13) including the exhaust gas (12),
**characterized in that** the testing device (30) includes
- a cell exposure device (31) being configured for accommodating at least one sample (1) of biological cells (2) and exposing the biological cells (2) to the test sample (13), and
- an analysing device (33) being configured for testing the biological cells (2).

2. Emission measurement system according to claim 1, wherein
- the sampling device (20) is arranged to be coupled with an exhaust output (14) of the combustion engine (11).

3. Emission measurement system according to one of the foregoing claims, wherein
- the sampling device (20) includes a metering device (22) being adapted for setting and supplying a predetermined test volume of the test sample (13).

4. Emission measurement system according to one of the foregoing claims, wherein
- the sampling device (20) includes a dilution device (23) being adapted for mixing the collected exhaust gas (12) with a test gas, in particular air, providing the test sample (13), and setting a predetermined concentration of the exhaust gas (12) in the test sample (13).

5. Emission measurement system according to one of the foregoing claims, wherein
- the sampling device (20) includes a temperature control unit (24) being adapted for setting a test temperature of the test sample (13) equal to a physiological temperature.

6. Emission measurement system according to one of the foregoing claims, wherein the cell exposure device (31) is configured for accommodating the at least one sample of the biological cells (2) including at least one of a cell culture and cell tissue, including at least one of
- stem cells, in particular induced pluripotent stem (ips) cells,
- differentiated ips cells, in particular ips lung cells and/or ips epithelial cells,
- a plurality of different genetic variants of the biological cells,
- 2D cell cultures,
- 3D cell cultures,
- cell organoids,
- primary lung cells, in particular from lung tissue taken from a human or an animal, and
- epithelial cells, in particular from epithelial tissue taken from a human or an animal.

7. Emission measurement system according to one of the foregoing claims, wherein
- the cell exposure device (31) is configured for accommodating the biological cells (2) in a frozen state and thawing the biological cells (2) before exposing them to the test sample (13).

8. Emission measurement system according to one of the foregoing claims, wherein the analysing device (33) has at least one of the features
- the analysing device (33) is configured for testing the biological cells (2) during and/or after exposing them to the test sample (13),
- the analysing device (33) is configured for measuring the interaction of particles with the biological cells (2), and
- the analysing device (33) is configured for detecting cell necrosis, cell apoptosis, miRNA pattern, epigenetic alterations, cell death, DNA breaks, miRNA expression, keeping a cell culture barrier function, cytokine release, ciliary activity (for airway epithelial cells), mucus viscosity alteration, mucus hypersecretion, cell metabolism, markers of oxidative stress, induction of an oxidative stress response and inflammatory response, induction of an integrated stress response, impairment of autophagy, impairment of epithelial antimicrobial defence, adsorption of test compounds, epithelial differentiation after repeated exposures, engineered reporter cell lines, e. g. iPSC single or multiple reporter lines.

9. Emission measurement system according to one of the foregoing claims, wherein
- the emission measurement system is a car test bench (110, 110A) being configured for a stationary test operation, and
- the car test bench (110, 110A) includes a roller module (111) being configured for accommodating a vehicle (10) including the combustion engine (11) to be tested.

10. Emission measurement system according to claim 9, including
- an exhaust gas container (27) being arranged for coupling with an exhaust output (14) of the combustion engine (11) of the vehicle (10), wherein
- the sampling device (20) is coupled with or provided by the exhaust gas container (27).

11. Emission measurement system according to one of the claims 1 to 8, wherein
- the emission measurement system is a portable test apparatus (120) being configured for a mobile test operation, and
- the portable test apparatus (120) includes a carrier device (121) being configured for coupling with a vehicle (10) including the combustion engine (11) to be tested.

12. Method of testing a combustion engine (11), in particular of a vehicle (10), comprising the steps of
- operating the combustion engine (11) and creating the exhaust gas (12),
- collecting the exhaust gas (12), and
- testing a test sample (13) including the exhaust gas (12),
**characterized in that**
- the testing step includes exposing at least one sample of biological cells (2) to the test sample (13) and testing the biological cells.

13. Method according to claim 12, wherein the collecting step includes at least one of
- collecting the exhaust gas (12) at an exhaust output (14) of the combustion engine (11) or in an exhaust gas container (27) coupled with an exhaust output (14) of the combustion engine (11),
- setting a test temperature of the test sample (13) equal to a physiological temperature,
- mixing the collected exhaust gas (12) with a test gas, in particular air, providing the test sample (13), and setting a predetermined concentration of the exhaust gas (12) in the test sample (13), and
- setting and supplying a predetermined test volume of the test sample (13).

14. Method according to one of the claims 12 and 13, wherein the biological cells (2) include at least one of a cell culture and cell tissue, including at least one of
- stem cells, in particular induced pluripotent stem (ips) cells,
- differentiated ips cells, in particular ips lung cells and/or ips epithelial cells,
- a plurality of different genetic variants of the biological cells,
- lung cells, in particular from lung tissue taken from a human or an animal, and
- epithelial cells, in particular from epithelial tissue taken from a human or an animal.

15. Method according to one of the claims 12 to 14, including the steps of
- providing the biological cells (2) in a frozen state, and
- thawing the biological cells (2) before exposing them to the test sample (13).

16. Method according to one of the claims 12 to 15, wherein the testing step includes at least one of the features
- testing the biological cells (2) is conducted during and/or after exposing them to the test sample (13),
- testing the biological cells (2) includes measuring the introduction of particles into the biological cells, and
- testing the biological cells (2) includes detecting cell necrosis, cell apoptosis, miRNA pattern, epigenetic alterations, cell death, DNA breaks, miRNA expression, keeping a cell culture barrier function, cytokine release, ciliary activity (for airway epithelial cells), mucus viscosity alteration, mucus hypersecretion, cell metabolism, markers of oxidative stress, induction of an oxidative stress response and inflammatory response, induction of an integrated stress response, impairment of autophagy, impairment of epithelial antimicrobial defence, adsorption of test compounds, epithelial differentiation after repeated exposures, engineered reporter cell lines, e. g. iPSC single or multiple reporter lines.

17. Method according to one of the claims 12 to 16, wherein
- the step of operating the combustion engine (11) includes setting of different operation phases of the combustion engine (11), and
- the steps of collecting the exhaust gas (12) and testing the test sample (13) are repeated in each of the operation phases of the combustion engine (11).

18. Method according to one of the claims 12 to 17, wherein
- an emission measurement system (100) is employed, which comprises the features of one of the claims 1 to 11.
